# EUROPEAN PATENT APPLICATION

(11) **EP 1 249 442 A1**
(43) Date of publication of application: **16.10.2002**
(21) Application number: 01900745.9
(22) Date of filing: 12.01.2001
(51) Int. Cl.: C07C 47/27, C07C 45/41, C07C 47/277, C07C 45/38, C07C 43/23, C07C 39/11, C07K 1/113, C07K 5/075, C07C 59/52, C07C 59/64

(54) **NOVEL ARYLPROPIONALDEHYDE DERIVATIVES AND PRODUCTION AND USE OF THE SAME**

(30) Priority: 20.01.2000 JP 2000011538
(71) Applicant: Ajinomoto Co., Inc., Tokyo 104-8315 (JP)
(72) Inventor: MORI, Kenichi Amino Sci. Lab., Ajinomoto Co. Inc., Kawasaki-shi Kanagawa-ken 210-0801 (JP); KAWAHARA, S. Amino Sci. Lab., Ajinomoto Co., Inc., Kawasaki-shi Kanagawa-ken 210-0801 (JP); TAKEMOTO, T. Amino Sci. Lab., Ajinomoto Co., Inc., Kawasaki-shi Kanagawa-ken 210-0801 (JP)
(74) Representative: Nicholls, Kathryn Margaret
(86) International application number: PCT/JP01/00174
(87) International publication number: WO 01/053243

(57) **Abstract**

3-(3-Methyl-4-hydroxyphenyl)-3-methylbutyl aldehyde is produced from 3-(3-methyl-4-hydroxyphenyl)-3-methylbutyric acid via a reaction for converting a carboxyl group into a formyl group. From such aldehyde derivative, via a reductive alkylation reaction with aspartame N-[N-[3-(3-methyl-4-hydroxyphenyl)-3-methylbutyl]-L-α-aspartyl]-L-phenylalanine 1-methyl ester useful as a sweetener having a high potency of sweetness can be derived easily and efficiently on an industrial production. Therefore, the derivative is extremely excellent as an intermediate for the production thereof.

The above butyric acid derivative is a novel compound, which can be easily produced by subjecting 3-(3-methyl-4-methoxyphenyl)-3-methylbutyric acid obtained in the reaction of 2-methoxytoluene with 3-methylcrotonic acid, to a reaction for converting a methoxy group into a hydroxyl group.

The present invention further provides several novel compounds useful as a production intermediate, such as the above aldehyde derivative and the like.

## Description

### Technical Field

The present invention relates to a novel arylpropyl aldehyde derivative useful as various production intermediates for a food product, a pharmaceutical product and the like, and in particular a production intermediate for N-[N-[3-(3-methyl-4-hydroxyphenyl)-3-methylbutyl]-L-α-aspartyl]-L-phenylalanine 1-methyl ester excellent as a sweetener having a high potency of sweetness, a process for production thereof, a use thereof (as a production intermediate), and novel production intermediates therefor.

### Background Art

In recent years, as eating habits have been improved to a high level, fatness caused by excessive sugar intake and diseases accompanied by fatness have been at issue. Accordingly, the development of a low-calory sweetener (sweetening agent) that replaces sugar has been strongly in demand. As a sweetener that is widely used at present, there is aspartame which is excellent in safety and quality of sweetness, and however, is somewhat problematic in stability.

Under these background, N-[N-[3-(3-methyl-4-hydroxyphenyl)-3-methylbutyl]-L-α-aspartyl]-L-phenylalanine 1-methyl ester has been found as a sweetener which is excellent in stability and moreover is better by far in degree of sweetness, i.e., has the advantage in cost per sweet taste, by a part of the present inventors and the like.

### Problem to be solved by Invention

As a process for production of N-[N-[3-(3-methyl-4-hydroxyphenyl)-3-methylbutyl]-L-α-aspartyl]-L-phenylalanine 1-methyl ester important as the sweetener described above, a process for alkylating reductively β-O-benzyl-α-L-aspartyl-L-phenylalanine methyl ester with 3-(3-methyl-4-benzyloxyphenyl)-3-methylbutyl aldehyde and NaB(OAc)₃H, followed by removing the benzyl group of a protecting group therefrom was thought up. However, 3-(3-methyl-4-benzyloxyphenyl)-3-methylbutyl aldehyde is synthesized through 7 stages of many reaction steps starting from the 3-methyl-4-hydroxyacetophenone, as shown in the following reaction process 1, and therefore it is difficult to say that the compound is an industrially profitable ground substance for reaction because of complication in the reaction process.

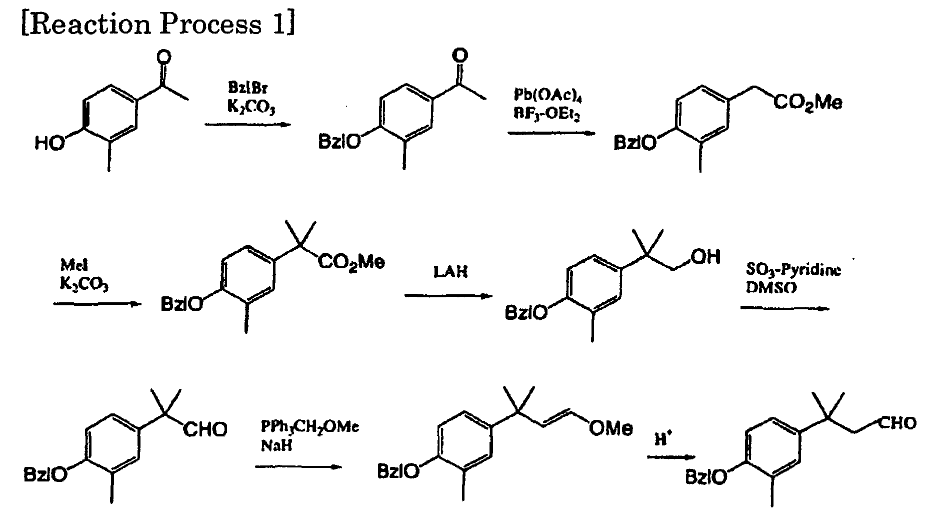

Under these above circumstances, it is requested to develop a process for producing industrially and simply the above aspartyl dipeptide ester derivative.

The problem to be solved by the present invention, is to provide a process for producing industrially and efficiently N-[N-[3-(3-methyl-4-hydroxyphenyl)-3-methylbutyl]-L- α-aspartyl]-L-phenylalanine 1-methyl ester excellent as a sweetener having a high potency of sweetness, and particularly a production intermediate therefor or a conventional process for the production thereof.

### Disclosure of Invention

The present inventors have earnestly investigated to solve the problem described above, and as a result succeeded in synthesizing newly an arylpropyl aldehyde derivative, specifically 3-(3-methyl-4-hydroxyphenyl)-3-methylbutyl aldehyde, and found that the compound is extremely excellent as the production intermediate for the sweetener having a high potency of sweetness described above. In addition, they have found one example preferable as an efficient process for production of this compound, as shown in the following reaction process 2. According to this process, the object compound can be synthesized through a reaction process having 3 stages totally, which comprises reacting 2-methoxytoluene with 3-methylcrotonic acid, preferably in the presence of an acid (first stage), converting the methoxy group in the butyric acid thus obtained to a hydroxyl group, preferably through an acid hydrolysis (second stage), and finally converting the carboxylic acid thus obtained to an aldehyde (third stage). Accordingly, with respect to the production of the production intermediate this process has the advantage (holds a dominant position) industrially in comparison with the process using as an production intermediate 3-(3-methyl-4-benzyloxyphenyl)-3-methylbutyl aldehyde which is complicate and requires 7 stages of many reaction steps as described above on producing the object compound. In addition, they have found that the compound also is industrially excellent as an intermediate for the production of N-[N-[3-(3-methyl-4-hydroxyphenyl)-3-methylbutyl]-L-α-aspartyl]-L-phenylalanine 1-methyl ester.

Above these various findings have led to the completion of the present invention.

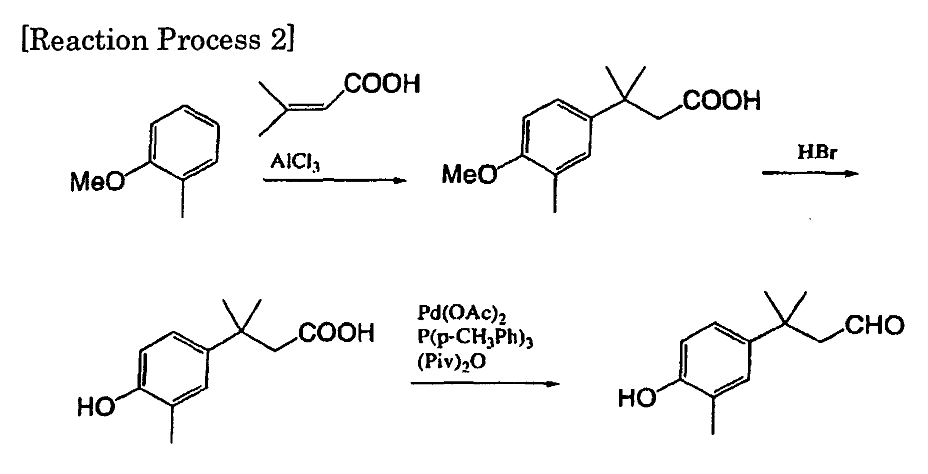

That is to say, in the present invention the following contents are contained.

### [1]

A process for producing 3-(3-methyl-4-hydroxyphenyl)-3-methylbutyl aldehyde, which comprises:
subjecting 3-(3-methyl-4-hydroxyphenyl)-3-methylbutyric acid to a reaction for converting a carboxyl group into a formyl group.

### [2]

The process as defined above, wherein in the above process, said 3-(3-methyl-4-hydroxyphenyl)-3-methylbutyric acid is obtained,
(a) by subjecting 3-(3-methyl-4-methoxyphenyl)-3-methylbutyric acid to a reaction for converting a methoxy group into a hydroxyl group; or
(b) by subjecting 3-(3-methyl-4-methoxyphenyl)-3-methylbutyric acid obtained through the reaction of 2-methoxytoluene with 3-methylcrotonic acid,
   to a reaction for converting a methoxy group into a hydroxyl group.

### [3]

The process as defined above, wherein in the above process, said reaction of 2-methoxytoluene with 3-methylcrotonic acid is conducted in the presence of an acid.

### [4]

The process as defined above, wherein in the above process, said reaction for converting a carboxyl group into a formyl group is any one of the following reactions:
(a) a reaction for converting a carboxylic acid to an aldehyde through reduction thereof and
(b) a reaction for converting a carboxyl group into a hydroxymethyl group, and thereafter converting the hydroxymethyl group into a formyl group.

### [5]

A process for producing N-[N-[3-(3-methyl-4-hydroxyphenyl)-3-methylbutyl]-L-α-aspartyl]-L-phenylalanine 1-methyl ester, which comprises:
subjecting 3-(3-methyl-4-hydroxyphenyl)-3-methylbutyl aldehyde and aspartame to a reductive alkylation reaction.

It is conventional and advantageous to use directly the 3-(3-methyl-4-hydroxyphenyl)-3-methylbutyl aldehyde as it is, produced in the above processes [1] to [4], for the starting material of the reductive alkylation reaction in this process [5]

### [6]

Novel benzene derivatives represented by the following general formula (1):

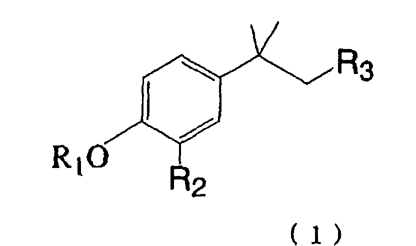

In the above formula, R₁ denotes a hydrogen atom or a lower alkyl group having 1 to 4 carbon atoms, R₂ denotes a lower alkyl group having 1 to 4 carbon atoms, and R₃ denotes any one of a carboxyl group, a formyl group and a hydroxymethyl group.

### [7]

Among the novel benzene derivatives as defined above, any one of the following compounds (a. to f.) is particularly preferable for the production intermediate:
a. 3-(3-methyl-4-hydroxyphenyl)-3-methylbutyl aldehyde;
b. 3-(3-methyl-4-methoxyphenyl)-3-methylbutyric acid;
c. 3-(3-methyl-4-hydroxyphenyl)-3-methylbutyric acid;
d. 3-(3-methyl-4-hydroxyphenyl)-3-methyl-1-butanol;
e. 3-(3-methyl-4-methoxyphenyl)-3-methylbutyl aldehyde; and
f. 3-(3-methyl-4-methoxyphenyl)-3-methyl-1-butanol.

### Embodiments for carrying out Invention

The embodiments for carrying out the present invention are explained in the followings.

The process for production of 3-(3-methyl-4-hydroxyphenyl)-3-methylbutyl aldehyde is explained in detail based on the above reaction process 2 by referring to preferable one example thereof at the center in the followings. However, since the example is shown as a preferable one, the present invention is not limited to the example unless the gist of the present invention is deviated from.

In case of conducting the reaction of 2-methoxytoluene with 3-methylcrotonic acid in the above first stage, it can be conducted without a solvent, and however it is conducted preferably in an organic solvent in the presence of an acid. As for the organic solvent, if employed, there is no special limitations thereto, as far as a material inactive to a ground substance for reaction, an acid and a reaction product may be employed therefor.

In case that an acid is employed, for the acid to be employed, a proton acid, such as sulfuric acid, p-toluenesulfonic acid and hydrogen chloride, or a Lewis acid, such as aluminum chloride and titanium tetrachloride, and the like, and any acids can be employed. Plural acids can be employed, respectively in the proton acid or Lewis acid, if necessary. Further, as the case may be, a proton acid can be employed in combination with a Lewis acid, such as combination of hydrogen chloride with aluminum chloride, if necessary. In addition, an acid fixed onto the surface of the solid phase is desirably employed, because a process for treatment may be simplified.

Further, an amount of acid to be employed is not limited particularly. In case that much excess of acid is employed to the 3-methylcrotonic acid, the reaction can be conducted in a shorter time to completion. From the economical point of view, preferably 5 molar equivalents or less, more preferably 3 molar equivalents or less, and further more preferably 0.1 to 3 molar equivalents or so, of the acid to the 3-methylcrotonic acid may be employed.

As for a quantity consumed (employed) of 2-methoxytoluene to the 3-methylcrotonic acid, it is not limited particularly. Preferably 0.5 molar equivalents or more, more preferably 1 molar equivalent or more, and further more preferably 1 to 10 molar equivalents or so, of 2-methoxytoluene to the 3-methylcrotonic acid may be employed.

As for a reaction temperature, there is no particular limitations thereto. The higher the reaction temperature is, the more the secondary reaction proceeds, and at a low temperature, the reaction speed becomes slow to an extreme. Accordingly, a temperature range of, preferably -20 to 180 °C or so, and more preferably -10 to 100 °C or so, and further more preferably 0 to 70 °C or so, is employed.

In case of conducting the reaction for converting a methoxy group into a hydroxyl group in the second stage from the 3-(3-methyl-4-methoxyphenyl)-3-methylbutyric acid obtained in the first stage, for example, 3-(3-methyl-4-methoxyphenyl)-3-methylbutyric acid is heated in an acidic aqueous solution to be able to convert the methoxy group into the hydroxyl group easily. For the acid, if employed, hydrogen chloride, hydrogen bromide, sulfuric acid and the like are cited. There is no particular limitations to the reaction temperature therefor. A temperature range of 80 to 150 °C or so is preferably employed.

In order to produce the aldehyde from 3-(3-methyl-4-hydroxyphenyl)-3-methylbutyric acid obtained in the above reaction, through the reaction for converting a carboxyl group into a formyl group in the third stage, the carboxylic acid is subjected directly to a reaction for reduction of the carboxyl group therein, whereby the objective 3-(3-methyl-4-hydroxyphenyl)-3-methylbutyl aldehyde can be obtained. In this case, preferably the compound can be reduced directly, based on the process described in Chemistry Letters, pp. 1143-1144, published in 1998, November, to be converted into 3-(3-methyl-4-hydroxyphenyl)-3-methylbutyl aldehyde as objective. This process is a process for reducing 3-(3-methyl-4-hydroxyphenyl)-3-methylbutyric acid with hydrogen in an organic solvent, with pivalic acid anhydride, palladium acetate and triphenylphosphine derivative added thereto. As for the organic solvent, there is no particular limitations thereto, as far as a solvent inactive to a ground substance (substrate) for reaction, a catalyst and a product may be employed therefor. For example, acetone, tetrahydrofuran (THF), toluene and the like are preferably employed. As for an amount employed of the pivalic acid anhydride, if employed, equimolecular or more of the compound to 3-(3-methyl-4-hydroxyphenyl)-3-methylbutyric acid, may be employed. For example, 1 to 5 moles or so of the compound to 1 mole of 3-(3-methyl-4-hydroxyphenyl)-3-methylbutyric acid may be preferably employed. As the triphenylphosphine derivative, if employed, triphenylphosphine and tritolylphosphine may be preferably employed. The palladium acetate and triphenylphosphine derivative are used as a catalyst, and therefore a few molar % or so thereof is sufficient for the quantities consumed thereof. As for a reaction temperature employed in the reaction, there is no particular limitations thereto. At a higher temperature, the reaction is promoted, and thus can be completed and finished in a short time. Accordingly, at a comparatively high temperature, it can be conducted at an advantage.

On the other hand, in place of the production of aldehyde through reduction from the carboxylic acid, as an another process, the carboxyl group therein is reduced completely to a hydroxymethyl group, and thereafter oxidized partially to be able to produce the above aldehyde derivative as objective. In this case, the above object compound can be easily produced, with the use of reduction - partial oxidation, which is known in itself (refer to Journal of Organic Chemistry, vol. 48, No. 25, 5043-5048, 1983).

Incidentally, in the production of the above aldehyde derivative, the process for converting a methoxy group into a hydroxyl group, firstly from 3-(3-methyl-4-methoxyphenyl)-3-methylbutyric acid, to produce 3-(3-methyl-4-hydroxyphenyl)-3-methylbutyric acid, followed by a reaction for leading the product to the aldehyde, was explained. Alternatively, the reaction for converting the methoxy group into the hydroxyl group can be conducted after the above reduction (formylation) of the butyric acid derivative or the reduction thereof (hydroxymethylation), in the same manner as above.

For example, through the above reduction (formylation) or the above reduction (hydroxymethylation) from 3-(3-methyl-4-methoxyphenyl)-3-methylbutyric acid, respectively in the same manner, 3-(3-methyl-4-methoxyphenyl)-3-methylbutyl aldehyde, or 3-(3-methyl-4-methoxyphenyl)-3-methyl-1-butanol can be also produced.

In the production of N-[N-[3-(3-methyl-4-hydroxyphenyl)-3-methylbutyl]-L-α-aspartyl]-L-phenylalanine 1-methyl ester from 3-(3-methyl-4-hydroxyphenyl)-3-methylbutyl aldehyde thus obtained, there is no particular difficulty. It can be easily produced by alkylating reductively this aldehyde with α-L-aspartyl-L-phenylalanine methyl ester (aspartame) under a condition for hydrogenation (hydrogen addition). Specifically, in the solvent which can dissolve the starting materials, for example, acetonitrile, acetic acid, ethyl acetate, alcohol, water-containing alcohol, or the like, in the presence of a catalyst for reductive alkyaltion, for example, a catalysts for hydrogenation such as palladium based catalyst, rhodium based catalyst and the like, a reductive alkylation reaction is conducted with hydrogen, more preferably, under suitable or effective reaction temperature and pressure to be able to produce the above object compound.

From the reaction mixture thus obtained, the catalyst used is removed, and the remaining material is subjected to purification step(s), such as a purification with a chromatography, a crystallization step and the like, if required, whereby a high purity of objective aspartyl dipeptide ester derivative of a sweetener having a high potency of sweetness described above can be separated.

### Examples

The present invention will be explained further in detail with reference to the following Examples therefor.

### (Example 1)

### Synthesis of 3-(3-methyl-4-methoxyphenyl)-3-methylbutyric acid

To 2-methoxytoluene (146.7 g, 1.2 mol), aluminum chloride (44 g, 0.33 mol) and 3-methylcrotonic acid (30 g, 0.3 mol) were added and the mixture was stirred under room temperature for 24 hours, and thereafter the reaction was stopped by the addition of six normal (6-N) hydrochloric acid aqueous solution (300 ml) thereto. Subsequently, from the thus obtained mixture the organic material was extracted with diethyl ether (150 ml). After that, to the organic layer one normal (1-N) caustic soda aqueous solution (100 ml) was added for extraction of the carboxylic acid(s). Thus obtained alkaline mixture was acidified by the addition of hydrochloric acid (HCl) aqueous solution, and the mixture was extracted with diethyl ether and the solvent therein was distilled off therefrom. Thus obtained residue was re-crystallized with hexane to obtain 3-(3-methyl-4-methoxyphenyl)-3-methylbutyric acid (39.6 g, yield: 59 %).
¹H NMR (CDCL₃) δ :1.43 (s, 6H), 2.21 (s, 3H), 2.61 (s, 2H), 3.80 (s, 3H), 6.74-6.77 (dd, 1H), 7.12-7.15 (m, 2H).

### (Example 2)

### Synthesis of 3-(3-methyl-4-hydroxyphenyl)-3-methylbutyric acid

3-(3-Methyl-4-methoxyphenyl)-3-methylbutyric acid (11.1 g, 0.05 mol), acetic acid (22.3 g) and 48 % hydrobromic acid aqueous solution (33.7 g, 0.2 mol) were mixed together, and stirred for 4 hours at 120 °C. The mixture was cooled to room temperature, and the thus obtained reaction solution was extracted with diethyl ether. The organic layer was washed with sodium chloride saturated aqueous solution, and then the solvent was distilled off therefrom to obtain 3-(3-methyl-4-hydroxyphenyl)-3-methylbutyric acid (10 g, yield: 95 %).
¹H NMR (CDCL₃) δ : 1.42 (s, 6H), 2.21 (s, 3H), 2.60 (s, 2H), 6.60-6.65 (dd, 1H), 6.99-7.10 (m, 2H).

### (Example 3)

### Synthesis of 3-(3-methyl-4-hydroxyphenyl)-3-methylbutyl aldehyde

Into a chemical reactor for hydrogen addition (hydrogenation) under elevated pressure, 3-(3-methyl-4-hydroxyphenyl)-3-methylbutyric acid (9.38 g, 0.045 mol), pivalic acid anhydride (16.8 g, 0.09 mol) and acetone (90 ml) were filled, and thereafter it was deaerated in the system of reaction.
Subsequently, a tetrahydrofuran (THF) solution (4.5 ml) of palladium acetate (101 mg, 0.45 mmol) and tri-p-tolylphosphine (685 mg, 2.25 mmol) produced previously, was added thereto and the mixture was stirred under hydrogen pressure of 5 MPa at 80 °C for 24 hours for reaction.

From the reaction solution, acetone was distilled off, and thus obtained residue was purified by the use of vacuum distillation to obtain 3-(3-methyl-4-hydroxyphenyl)-3-methylbutyl aldehyde (4.82 g, yield: 56 %).
Boiling point (b. p.): 136 °C (1 mmHg[133Pa]).
¹H NMR (CDCL₃) δ : 1.42 (s, 6H), 2.24 (s, 3H), 2.62 (d, 2H), 5.13 (s, 1H), 6.70 - 6.73 (dd, 1H), 7.03-7.11 (m, 2H), 9.49 (t, 1H).

### (Example 4)

### Synthesis of N-[N-[3-(3-methyl-4-hydroxyphenyl)-3-methylbutyl]-L-α-aspartyl]-L-phenylalanine 1-methyl ester

Aspartame (5.89 g, 20.0 mmol) and 3-(3-methyl-4-hydroxyphenyl)-3-methylbutyl aldehyde (3.202 g, 16.7 mmol) were added to 80 % methanol aqueous solution (200 ml), and the mixture was stirred at 40 °C in a short time. To this solution, 10 % palladium on activated carbon in the water content of 50 % (1.78 g) was added, and the mixture was stirred under hydrogen atmosphere of normal pressure (0.1 MPa) at 25 °C for 40 hours. Thus obtained reaction solution was filtrated to remove the catalyst, and the filtrate was subjected to the high performance liquid chromatography (HPLC) for determination and thereby the title compound (7.06 g, 15.03 mmol, 90 %) was produced.

### Effect of Invention

By using the novel 3-(3-methyl-4-hydroxyphenyl)-3-methylbutyl aldehyde used as the production intermediate in the present invention, in a reductive alkylation reaction with aspartame, N-[N-[3-(3-methyl-4-hydroxyphenyl)-3-methylbutyl]-L- α-aspartyl]-L-phenylalanine 1-methyl ester useful as a sweetener having a high potency of sweetness, can be produced easily and efficiently on an industrial scale.

The aldehyde derivative described above is a novel compound, and can be produced easily and efficiently in a process for subjecting 3-(3-methyl-4-methoxyphenyl)-3-methylbutyric acid obtained through the reaction of 2-methoxytoluene with 3-methylcrotonic acid, to a reaction for converting a methoxy group into a hydroxyl group to produce a carboxylic acid, followed by a reaction for converting a carboxyl group of the carboxylic acid thus obtained into a formyl group. Therefore, according to the present invention, the sweetener having a high potency of sweetness described above can be produced advantageously on an industrial scale.

Also, there are provided several novel compounds useful as a production intermediate, such as the aldehyde derivative described above and the like.

## Claims

1. A process for producing 3-(3-methyl-4-hydroxyphenyl)-3-methylbutyl aldehyde, which comprises:
subjecting 3-(3-methyl-4-hydroxyphenyl)-3-methylbutyric acid to a reaction for converting a carboxyl group into a formyl group.

2. The process as defined in claim 1, wherein said 3-(3-methyl-4-hydroxyphenyl)-3-methylbutyric acid is obtained,
a. by subjecting 3-(3-methyl-4-methoxyphenyl)-3-methylbutyric acid to a reaction for converting a methoxy group into a hydroxyl group; or
b. by subjecting 3-(3-methyl-4-methoxyphenyl)-3-methylbutyric acid obtained through the reaction of 2-methoxytoluene with 3-methylcrotonic acid,
to a reaction for converting a methoxy group into a hydroxyl group.

3. The process as defined in claim 2, wherein said reaction of 2-methoxytoluene with 3-methylcrotonic acid is conducted in the presence of an acid.

4. The process as defined in claim 1, wherein said reaction for converting a carboxyl group into a formyl group is any one of the following reactions:
a. a reaction for converting a carboxylic acid to an aldehyde through reduction thereof and
b. a reaction for converting a carboxyl group into a hydroxymethyl group, and thereafter converting the hydroxymethyl group into a formyl group.

5. A process for producing N-[N-[3-(3-methyl-4-hydroxyphenyl)-3-methylbutyl]-L- α-aspartyl]-L-phenylalanine 1-methyl ester, which comprises:
subjecting 3-(3-methyl-4-hydroxyphenyl)-3-methylbutyl aldehyde and aspartame to a reductive alkylation reaction.

6. The process as defined in any one of claims 1 to 4, which further comprises:
subjecting the 3-(3-methyl-4-hydroxyphenyl)-3-methylbutyl aldehyde thus produced to a reductive alkylation reaction with aspartame to form N-[N-[3-(3-methyl-4-hydroxyphenyl)-3-methylbutyl]-L- α-aspartyl]-L-phenylalanine 1-methyl ester.

7. A benzene derivative represented by the following general formula (1): 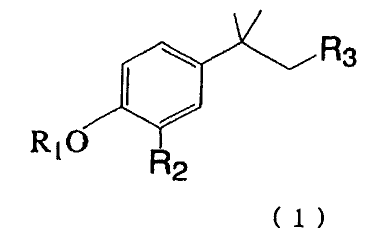 In the above formula, R₁ denotes a hydrogen atom or a lower alkyl group having 1 to 4 carbon atoms, R2 denotes a lower alkyl group having 1 to 4 carbon atoms, and R₃ denotes any one of a carboxyl group, a formyl group and a hydroxymethyl group.

8. The benzene derivative as defined in claim 7, which is any one of the following compounds a. to f.:
a. 3-(3-methyl-4-hydroxyphenyl)-3-methylbutyl aldehyde;
b. 3-(3-methyl-4-methoxyphenyl)-3-methylbutyric acid;
c. 3-(3-methyl-4-hydroxyphenyl)-3-methylbutyric acid;
d. 3-(3-methyl-4-hydroxyphenyl)-3-methyl-1-butanol;
e. 3-(3-methyl-4-methoxyphenyl)-3-methylbutyl aldehyde; and
f. 3-(3-methyl-4-methoxyphenyl)-3-methyl-1-butanol.
